# EUROPEAN PATENT APPLICATION

(11) **EP 2 409 648 A1**
(43) Date of publication of application: **25.01.2012**
(21) Application number: 11163287.3
(22) Date of filing: 20.04.2011
(51) Int. Cl.: A61B 8/06, A61B 8/08, A61B 8/14

(54) **Ultrasonic diagnostic apparatus and method thereof**

(30) Priority: 19.07.2010 KR 20100069393
(71) Applicant: Samsung Medison Co., Ltd., Nam-myun Hongchun-gun Kangwon do 250-875 (KR)
(72) Inventor: Lee, Jin Yong, Seoul (KR); Shim, Jae Yoon, Seoul (KR)
(74) Representative: Schmid, Wolfgang

(57) **Abstract**

The present disclosure relates to an ultrasonic diagnostic apparatus for providing a combination of an ultrasound image and a color flow image. The ultrasonic diagnostic apparatus detects a color index of each of pixels in a color flow image and provides a contour image composed of contour lines. Here, each of the contour lines is formed by grouping and connecting pixels having the same color index based on the detected color indexes of the respective pixels and formed for at least one color index level. A diagnosis method of the apparatus is also disclosed.

## Description

### TECHNICAL FIELD

The present disclosure relates to an ultrasonic diagnostic apparatus and a diagnosis method thereof. More particularly, the present disclosure relates to an ultrasonic diagnostic apparatus and a diagnosis method thereof, which facilitates identification of a distribution and change of blood flow rate in an object on a part-by-part basis in providing a combination of an ultrasound image and a color flow image by ultrasonic diagnostic apparatus.

### BACKGROUND

Ultrasonic diagnostic apparatuses are used in a wide range of applications. For example, ultrasonic diagnostic apparatuses are used in a wide range of medical applications due to non-invasive and non-destructive characteristics thereof. Recently, high performance ultrasonic diagnostic apparatuses have been used to provide two-dimensional or three-dimensional images of internal organs.

Generally, a probe of the ultrasonic diagnostic apparatus includes a transducer for transmitting and receiving wideband ultrasound signals. When the transducer is electrically driven, an ultrasound signal is generated by the transducer and delivered to an object. An ultrasound wave echo signal, which is reflected from the object to the transducer, is transformed into an electrical signal. Then, the transformed electrical signal is amplified and processed to provide ultrasound image data.

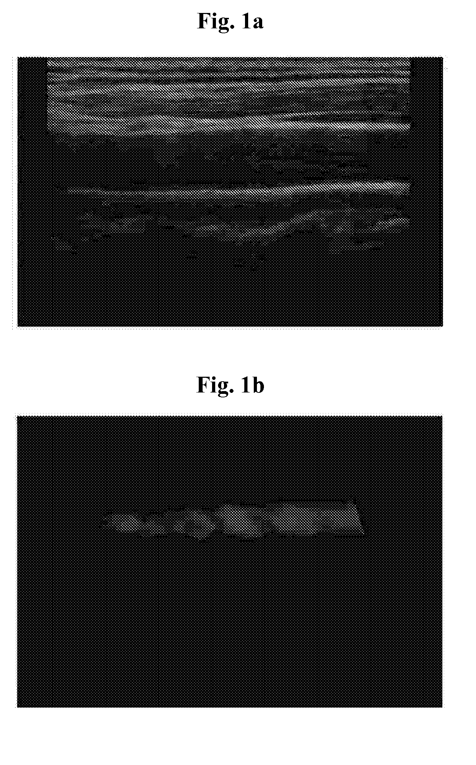

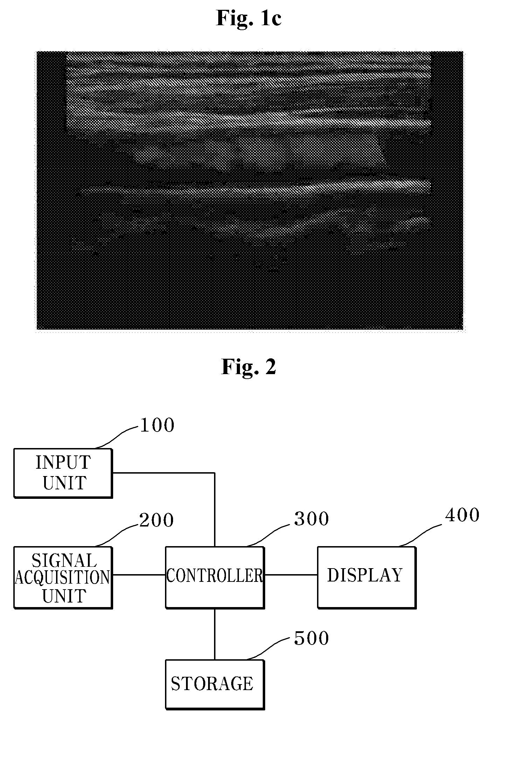

Meanwhile, the ultrasonic diagnostic apparatus utilizes the Doppler effect to generate a color flow image, which indicates moving rates of an object and a scatterer. Fig. 1a illustrates a B-mode image, Fig. 1b illustrates a color flow image, and Fig.1c illustrates a combination of the B-mode image and the color flow image. The ultrasonic diagnostic apparatus generates a color flow image, as shown in Fig. 1b, for a designated particular range based on Doppler data, and then combines this color flow image with the B-mode image to form and display an image, as shown in Fig. 1c. Herein, the term "B-mode" refers to a diagnostic mode in which magnitude of ultrasound echo signals reflected from an object is represented based upon brightness.

In the color flow image, a red tone represents a blood flow approaching the transducer side, whereas a blue tone represents another blood flow moving away from the transducer side. Colors in the color flow image represent the rate of the blood flow. For example, the darker the red or blue tone, the slower the blood flow rate, whereas the brighter the red or blue tone, the faster the blood flow rate.

In a conventional ultrasonic diagnostic apparatus, a change in rate from one region to another region of an object can be represented by the image as shown in Fig. 1c. However, when the rate change is very slow, it is difficult not only to recognize such a change, but also to identify a distribution and change of the blood flow rate in the object on part-by-part basis. Moreover, it is difficult to identify a distribution of regions having similar blood flow rates in the object.

Therefore, there is a need for an improved ultrasonic diagnostic apparatus that overcomes such problems.

### SUMMARY

The present disclosure provides an improved ultrasonic diagnostic apparatus, which can facilitate identification of a distribution and change of blood flow rate in an object on a part-by-part basis when providing a combination of an ultrasound image and a color flow image, and an improved ultrasonic diagnosis method thereof.

In accordance with one aspect, an ultrasonic diagnostic apparatus for providing a combination of an ultrasound image and a color flow image detects a color index of each of pixels in a color flow image and provides a contour image composed of contour lines. Here, each of the contour lines is on a level-by-level basis formed by grouping and connecting pixels having the same color index based on the detected color indexes of the respective pixels and formed for at least one color index level.

The ultrasonic diagnostic apparatus includes: a signal acquisition unit that acquires an ultrasound signal reflected from an object and a Doppler signal with respect to a particular portion of the object; a controller that generates an ultrasound image signal and a color flow image signal based on the ultrasound signal and the Doppler signal, detects a color index of each of pixels in the color flow image, and provides a contour image composed of contour lines, each of the contour lines being formed by grouping and connecting pixels having the same color index and being formed for at least one color index level; and a display that displays the ultrasound image signal, the color flow image signal, and the contour image signal.

The ultrasound image may be a B-mode image.

The color index of each of the pixels may correspond to a blood flow rate in the corresponding pixel.

The number of color index levels may be arbitrarily set and the contour lines may be formed corresponding to the number of color index levels.

The controller may calculate and provide an area corresponding to a region set by a particular contour selected from the contour lines.

In accordance with another aspect, a diagnosis method of an ultrasonic diagnostic apparatus includes: generating an ultrasound image signal and a color flow image signal; detecting a color index of each of pixels in the color flow image signal; grouping pixels having the same color index based on the detected color indexes of the respective pixels; and forming a contour image composed of contour lines. Each of the contour lines is formed by connecting the grouped pixels and provided for at least one color index level.

The method may further include receiving a selection signal of a particular contour line selected among the contour lines each being formed for the at least one color index level; and calculating and providing an area corresponding to a region designated by the selected particular contour line.

### BRIEF DESCRIPTION OF THE DRAWINGS

The figures depict one or more implementations in accordance with the present disclosure, by way of example only, not by way of limitation. In the figures, like reference numerals refer to the same or similar elements.

Fig. 1a illustrates a B-mode image;

Fig. 1b illustrates a color flow image;

Fig. 1c illustrates a combination of the B-mode image and the color flow image;

Fig. 2 is a block diagram of an ultrasonic diagnostic apparatus in accordance with an exemplary embodiment of the present disclosure;

Fig. 3 is a flowchart of a method of forming a contour image in the ultrasonic diagnostic apparatus in accordance with the embodiment of the present disclosure;

Fig. 4a illustrates one example of a color flow image;

Fig. 4b illustrates the contour image with respect to the color flow image of Fig. 4a; and

Figs. 5a to 5c illustrate contour images that are generated corresponding to the number of color index levels.

### DETAILED DESCRIPTION

Exemplary embodiments of the present disclosure will now be described in detail with reference to the accompanying drawings. It should be noted that the drawings are not to precise scale and may be exaggerated in thickness of lines or size of components for descriptive convenience and clarity. Furthermore, terms used herein are defined by taking functions of the present disclosure into account and can be changed according to the custom or intention of users or operators. Therefore, the terms should be defined according to the overall disclosures set forth herein.

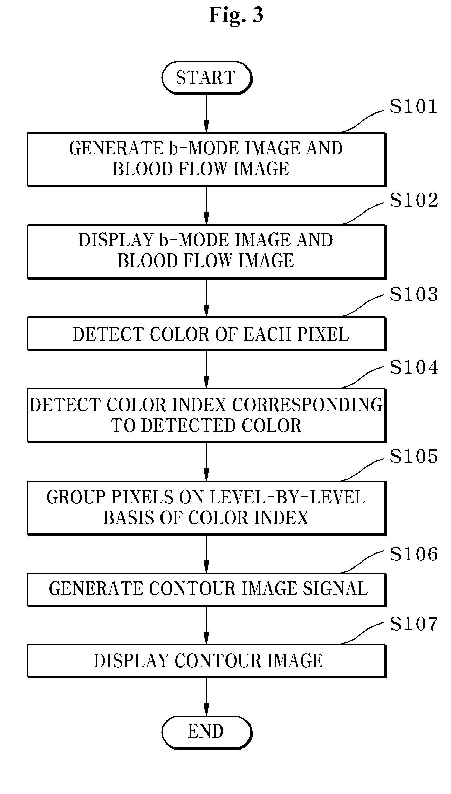

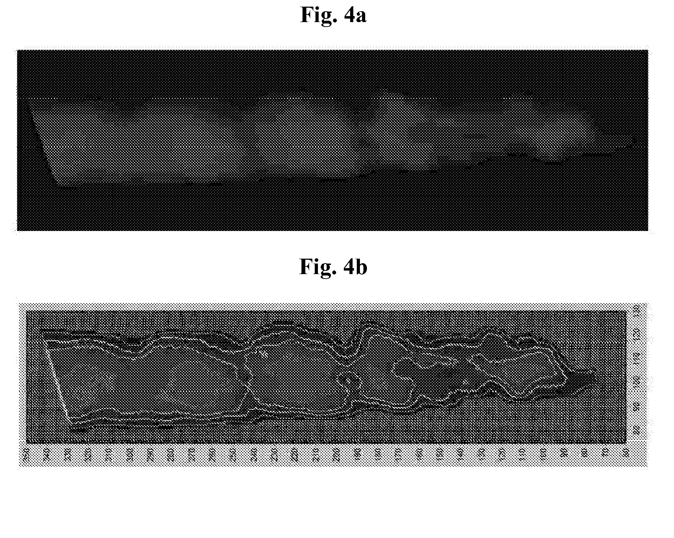

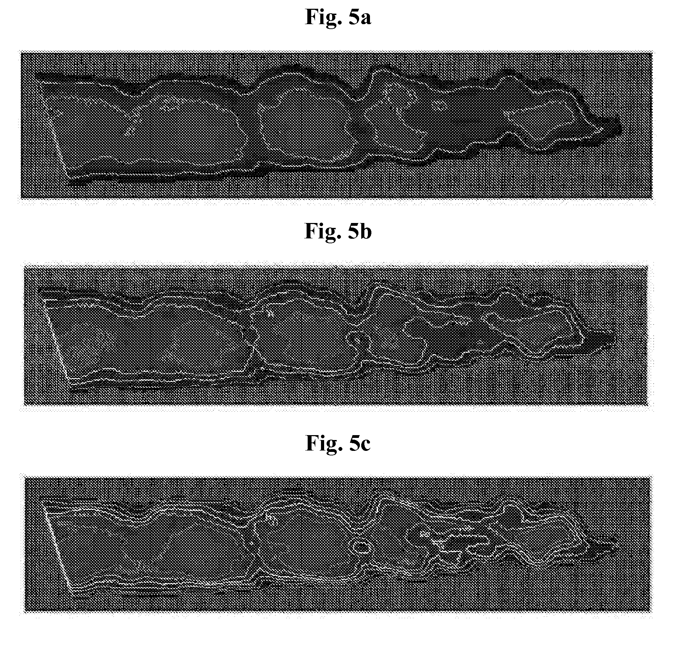

Fig. 2 is a block diagram of an ultrasonic diagnostic apparatus in accordance with an exemplary embodiment of the present disclosure. Fig. 3 is a flowchart of a method of forming a contour image in the ultrasonic diagnostic apparatus in accordance with the embodiment of the present disclosure. Fig. 4a illustrates one example of a color flow image and Fig. 4b illustrates the contour image with respect to the color flow image of Fig. 4a. Figs. 5a to 5c illustrate contour images that are generated corresponding to the number of color index levels.

Referring to Fig. 2, the ultrasonic diagnostic apparatus according to the exemplary embodiment includes a signal acquisition unit 200, a controller 300, and a display 400. The signal acquisition unit 200 acquires an ultrasound signal reflected from an object and a Doppler signal with respect to a particular portion of the object. The controller 300 generates an ultrasound image signal and a color flow image signal based on the ultrasound signal and the Doppler signal, detects a color index of each of pixels in the color flow image, and provides a contour image composed of contour lines. Here, each of the contour lines is formed by grouping and connecting pixels having the same color index and formed for at least one color index level. The display 400 displays the ultrasound image signal, the color flow image signal, and the contour image signal.

The ultrasonic diagnostic apparatus according to the embodiment will be described with reference to Fig. 2 to Fig. 5c.

The ultrasonic diagnostic apparatus starts operation when a user inputs various information and conditions for acquiring an ultrasound image via an input unit 100.

First, the signal acquisition unit 200 sends an ultrasound signal to an object and receives an echo ultrasound signal reflected from the object to form an ultrasound image. Further, the signal acquisition unit 200 acquires a Doppler signal with respect to a certain region designated by a user via the input unit 100. Here, the storage 500 may store the ultrasound signal, the Doppler signal and preset information.

Then, the controller 300 generates an ultrasound image signal of a particular mode, for example, a B-mode image signal, based on the ultrasound signal from the signal acquisition unit 200, and generates a color flow image signal based on the Doppler signal (in S101). The display 400 displays a B-mode image and a color flow image in response to the ultrasound signal and the color flow image signal (in S102).

Next, when a user selects a contour image mode through the input unit 100, the controller 300 detects a color of each of the pixels in the color flow image as shown in Fig. 4a (in S103) and then detects a color index corresponding to each of the detected colors (in S104). Herein, the color indexes are indicated by preset color scales (for example, C₀ ∼ C₂₅₅ or C₀∼ C₅₁₁), which are obtained by dividing a red or blue color of each pixel from the most dark tone to the most bright tone in order of brightness in the color flow image, wherein a color index of each of pixels corresponds to a blood flow rate in the corresponding pixel.

Further, the term "contour image mode" refers to a mode of displaying a contour image that is composed of contour lines, each of which is formed by grouping and connecting pixels having the same color index and is provided for at least one color index level. This contour image mode will be explained in more detail below.

Then, the controller 300 groups pixels having the same color index based on the detected color indexes of the respective pixels for each of the color index levels (in S105). That is, the controller 300 groups pixels having the same color index for each of the color index levels preset or predetermined by a user.

Then, the controller 300 connects the grouped pixels to generate a contour line for each of the color index level so that a contour image signal can be formed from the contour lines (in S106). In other words, the controller 300 connects the grouped pixels having the same color index to generate the contour image signal such that the contour image can be displayed, as shown in Fig. 4b (in S107).

In the above, the number of color index levels can be arbitrarily set by a user and the contour lines are formed corresponding to the number of color index levels. For example, if a user sets three distinguishable color index levels, three contour lines having a constant color index separation therebetween are formed, as shown in Fig. 5a. When a user increases the number of color index levels, for example, to 5 or 8 in order to apprehend a distribution of blood flow rates in more detail, more detailed contour images can be obtained, as shown in Figs. 5b and 5c.

As such, the ultrasonic diagnostic apparatus detects the color indexes corresponding to the blood flow rate in the color flow image signal on a pixel-by-pixel basis, groups pixels having the same color index based on the detected color indexes, and connects the grouped pixels, thereby producing contour lines for at least one of the color index levels. Then, a contour image is formed using the contour lines. This contour image allows a user to easily and precisely identify the distribution and change of the blood flow rate in the object on a part-by-part basis. Further, according to the embodiment, when a plurality of contour lines having a constant color index distance therebetween is formed, it can be easily apprehended by evaluating proximity between the contour lines whether the change of the blood flow rate is rapid or smooth in a certain part of an object.

In this embodiment, the color index is illustrated as being detected after the B-mode image and the color flow image are displayed. However, the resultant image can be displayed immediately after forming the contour image without displaying the B-mode image and the color flow image therebetween in accordance with user selection and setting. Further, although this embodiment describes to the controller 300 as generating all of an ultrasound image signal such as a B-mode image, a color flow image signal, and a contour image signal, it should be understood that each of these signals may be generated by a separate controller in another embodiment, which also falls within the scope of the controller 300 according to the present disclosure.

On the other hand, the ultrasonic diagnostic apparatus further includes a function of calculating an area of a region constituted by specific contour lines among the grouped contour lines, which are formed by connecting pixels having the same blood flow rate or the same color index.

A selection signal of a particular contour line among the contour lines each formed for at least one color index level is input to the ultrasonic diagnostic apparatus through the input unit 100. Then, the controller 300 calculates and provides an area corresponding to the region designated by the selected contour line. This calculation can be implemented by various methods adopted by existing image processing applications.

By doing this operation, a user can apprehend not only an area occupied by a particular region representing a certain blood flow rate in an object, but also an amount of blood flow in the corresponding region. Further, this operation enables diagnosis and identification of regurgitation or internal bleeding, for example, in the heart or in a particular region of the human body. Particularly, this operation can be effectively used for measuring the amount of regurgitation from the valve of the heart.

According to the embodiments, the ultrasonic diagnostic apparatus and the diagnosis method thereof may facilitate not only identification of a distribution and change of blood flow rate in an object on part-by-part basis, but also diagnosis of a blood regurgitation or internal bleeding part of the object, in providing a combination of an ultrasound image and a color flow image.

While the foregoing has described what are considered to be the best mode and/or other examples, it is understood that various modifications may be made therein and that the subject matter disclosed herein may be implemented in a variety of different ways, and that the present disclosure may be applied to numerous applications, only some of which have been described herein. The following claims should be considered to cover all applications, modifications and variations within the true scope of the present disclosure.

## Claims

1. An ultrasonic diagnostic apparatus for providing a combination of an ultrasound image and a color flow image,
**characterized in that**:
the ultrasonic diagnostic apparatus detects a color index of each of pixels in a color flow image, and provides a contour image composed of contour lines, each of the contour lines being formed by grouping and connecting pixels having the same color index based on the detected color indexes of the respective pixels and formed for at least one color index level.

2. The apparatus of claim 1, **characterized by** comprising:
a signal acquisition unit configured to acquire an ultrasound signal reflected from an object and a Doppler signal with respect to a particular portion of the object;
a controller configured to generate an ultrasound image signal and a color flow image signal based on the ultrasound signal and the Doppler signal, detect a color index of each of pixels in the color flow image, and provide a contour image composed of contour lines, each of the contour lines being formed by grouping and connecting pixels having the same color index and being formed for at least one color index level; and
a display configured to display the ultrasound image signal, the color flow image signal, and the contour image signal.

3. The apparatus of claim 1 or 2, **characterized in that** the ultrasound image is a B-mode image.

4. The apparatus of claim 1 or 2, **characterized in that** the color index of each of the pixels corresponds to a blood flow rate in the corresponding pixel.

5. The apparatus of claim 1 or 2, **characterized in that** the number of color index levels is arbitrarily set and the contour lines are formed corresponding to the number of color index levels.

6. The apparatus of claim 1 or 2, **characterized in that** the controller calculates and provides an area corresponding to a region designated by the selected particular contour.

7. A diagnosis method of an ultrasonic diagnostic apparatus, **characterized by** comprising:
generating an ultrasound image signal and a color flow image signal;
detecting a color index of each of pixels in the color flow image signal;
grouping pixels having the same color index based on the detected color indexes of the respective pixels; and
forming a contour image composed of contour lines, each of the contour lines being formed by connecting the grouped pixels and formed for at least one color index level.

8. The method of claim 7, **characterized in that** the color index of each of the pixels corresponds to a blood flow rate in the corresponding pixel.

9. The method of claim 7, **characterized in that** the number of color index levels is arbitrarily set and the contour lines are formed corresponding to the number of color index levels.

10. The method of clam 7, **characterized by** further comprising:
receiving a selection signal of a particular contour line selected among the contour lines each being formed for the at least one color index level; and
calculating and providing an area corresponding to a region designated by the selected particular contour line.
